# EUROPÄISCHE PATENTSCHRIFT

(11) **EP 2 354 771 B1**
(45) Veröffentlichungstag und Bekanntmachung des Hinweises auf die Patenterteilung: **26.06.2019**
(21) Anmeldenummer: 11153930.0
(22) Anmeldetag: 09.02.2011
(51) Int. Cl.: G01G 19/50, A61B 5/053

(54) **Körperanalysewaage**
Body analysis scales
Balance d'analyse de corps

(30) Priorität: 09.02.2010 DE 102010007448
(43) Veröffentlichungstag der Anmeldung: 10.08.2011
(73) Patentinhaber: Leifheit AG, 56377 Nassau (DE)
(72) Erfinder: Denk, Andre, 45131, Essen (DE); Emter, Artjom, 56077, Koblenz (DE); Moddick, Christian, 48317, Drensteinfurth (DE)
(74) Vertreter: Bungartz Christophersen Partnerschaft mbB Patentanwälte

(56) Entgegenhaltungen:
- EP-A1- 1 329 190
- EP-A1- 1 576 922
- WO-A1-2009/152624
- JP-A- 2003 169 784
- US-A1- 2004 171 962
- US-A1- 2005 113 712
- US-A1- 2009 105 557

## Beschreibung

### Technisches Gebiet

Die Erfindung betrifft eine Körperanalysewaage zum Messen von Körperwerten, insbesondere Körperfettwerten, einer Person , mit einer als Wägeplatte dienenden Kontaktplatte, auf der die Person während einer Körperwertmessung steht, zwei nebeneinander angeordneten, von der Person insbesondere durch Aufstellen einer Fußsohle zu berührenden Messelementen zur Messung einer zumindest mit einem Körperwert korrelierenden Größe einer Auswerteelektronik, die aus der Größe den zu bestimmenden Körperwert zu berechnen vermag und einer Anzeigevorrichtung zur Anzeige des berechneten Körperwerts.

### Stand der Technik

Aus dem Stand der Technik sind Messgeräte zum Messen von Körperwerten, wie beispielsweise Körperanalysewaagen, seit langem bekannt. Die bekanntesten Körperanalysewaagen sind die seit einigen Jahren stark am Markt vertretenen Körperfettmesswaagen. Diese Personenwaagen messen neben dem Körpergewicht über auf der Trittplatte der Waage angeordnete Elektroden auch die bioelektrische Impedanz der die Waage benutzenden Person. Dabei sind mehrere Messfelder vorgesehen, die zur Ermittlung der Messwerte gezielt mit den Füßen betreten werden müssen. Diese Messfelder weisen Elektroden auf, auf die zu analysierende Person treten muss, damit ein Messvorgang erfolgen kann. Eine derartig ausgebildete Körperanalysewaage wird beispielsweise in DE 102 05 822, JP 2003 169784 und US 2005/113712 beschrieben.

Ferner sind tragbare Messgeräte zum Messen von Körperwerten insbesondere von dem Körperfettanteil der zu analysierenden Person bekannt. In diesen Messgeräten erfolgt der Messvorgang indem die zu analysierende Person mit den Fingern die Elektroden kontaktiert, die auf dem Messgerät vorgesehen sind. Ein derartiges Messgerät ist aus DE 698 16 411 T2 bekannt.

In den bekannten Messgeräten weist die Anbringung der Elektroden an der Seite, die zu der analysierenden Person weist, eine Vielzahl von Nachteilen auf. So bestehen die Elektroden vorwiegend aus Metall und weisen Kanten auf, an denen sich die zu analysierende Person verletzen kann. Außerdem beschränkt die Anbringung der Elektroden an der zur analysierenden Person weisenden Seite die Designmöglichkeiten der Körperanalysewaage. Ferner unterliegen die Elektroden äußeren Einflüssen, die z.B. aufgrund Oxidation einen Verschleiß der Elektroden bewirken. Die Elektroden werden auch sehr rutschig, wenn sie nass sind. Ein weiterer Nachteil der bekannten Messgeräte besteht darin, dass sich manche Materialien für die Elektroden aufgrund ihrer technischen Eigenschaften für den Messvorgang sehr gut eignen, diese Materialien aber nicht in Kontakt mit Haut gebracht werden dürfen und somit in dem Messgerät nicht verwendet werden können. Da bei einem Messvorgang die zu analysierende Person barfuß direkt auf die Elektroden tritt, empfindet die zu analysierende Person den Messvorgang als unangenehm, weil die Elektroden als kalt empfunden werden. Des Weiteren ist die Reinigung des Messgeräts schwierig, weil die Elektroden eine ungleichmäßige Oberfläche besitzen. Letztendlich besteht auch ein Nachteil der bekannten Messgeräte darin, dass ein Gleichstrom das Messsignal verfälschen kann.

### Kurzbeschreibung der Erfindung

Es ist die Aufgabe der vorliegenden Erfindung, ein Messgerät bereitzustellen, dass die oben genannten Nachteile löst.

Die Aufgabe wird durch den Patentanspruch 1 gelöst. Vorteilhafte Ausgestaltungen der Erfindung werden in den Unteransprüchen genannt.

Die Erfindung weist den Vorteil auf, dass zumindest ein Teil der Kontaktplatte zwischen der zu analysierenden Person und dem Messelement vorgesehen ist. Bei einer Anordnung des Messelements, so dass dieses während nicht in direkten Kontakt mit der zu analysierenden Person steht, treten die oben genannten Nachteile nicht auf. Ergänzend wird erwähnt, dass in dem erfindungsgemäßen Messgerät eine Verfälschung des Messsignals durch einen Gleichstrom aufgrund einer galvanischen Trennung des Messelements von der zu analysierenden Person nicht auftreten kann.

Eine weitere vorteilhafte Ausgestaltung der Erfindung besteht darin, dass das Messelement innerhalb der Kontaktplatte oder auf einer zu der analysierenden Person entfernten Seite der Kontaktplatte angeordnet ist. Somit ergeben sich unterschiedliche Designmöglichkeiten des Messgeräts.

### Kurze Beschreibung der Zeichnungsfiguren

In den Zeichnungen ist der Erfindungsgegenstand schematisch dargestellt und wird anhand der Figuren nachfolgend beschrieben, wobei gleich wirkende Elemente mit denselben Bezugszeichen versehen sind. Dabei zeigen:
Fig. 1 eine Draufsicht auf eine Körperanalysewaage,
Fig. 2 eine Seitenansicht der Körperanalysewaage und einen Teil der zu analysierenden Person, der mit der Körperanalysewaage während des Messvorgangs in Kontakt steht,
Fig. 3 ein elektrisches Ersatzschaltbild der in Figur 2 gezeigten Körperanalysewaage.

### Beschreibung der Ausführungsarten

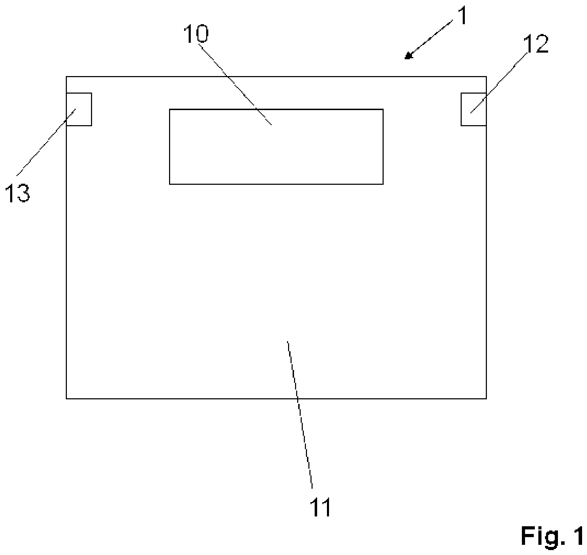

Das in Fig. 1 gezeigte Messgerät ist als eine Körperanalysewaage 1 ausgebildet und weist eine Kontaktplatte 11 auf. Die Erfindung ist durch den unabhängigen Anspruch definiert. Die Kontaktplatte 11 entspricht einer Platte, auf die sich die zu wiegende Person zum Wiegen und zum Ermitteln der Körperanalysewerte stellt. Unterhalb der Kontaktplatte 11 sind in Fig. 1 nicht gezeigte Messelemente vorgesehen, mittels denen die Körperanalysewerte ermittelt werden.

Zusätzlich weist die Körperanalysewaage 1 eine Sende- und Empfangseinheit 13 auf, die die gemessenen Werte an weitere in der Figur 1 nicht dargestellte Vorrichtungen sendet bzw. Informationen von einer externen an der Körperanalysewaage 1 nicht vorgesehenen Sendevorrichtung empfängt. Die Datenübertragung zwischen der externen Sendevorrichtung und der an der Körperanalysewaage 1 angeordneten Empfangseinheit kann über Funk oder über Infrarot erfolgen. Bei der externen Sendevorrichtung kann es sich auch um eine Sendevorrichtung handeln, die mit dem Internet verbunden ist. Die Datenübertragung kann in diesem Fall über WLAN erfolgen.

Die Sende- und Empfangseinheit 13 ist in Fig. 1 als ein einziges Bauteil dargestellt. Es sind Ausführungen der Körperanalysewaage 1 vorstellbar, bei denen die Sende- und Empfangseinheit 13 als getrennte Bauteile ausgebildet sind. Die Sende- und Empfangseinheit 13 können als Antennen ausgebildet sein.

Ferner weist die Körperanalysewaage 1 eine in der Kontaktplatte 11 befindliche Anzeigevorrichtung 10 auf. Die Anzeigevorrichtung 10 dient zum Anzeigen der mittels der Körperanalysewaage 1 gemessenen Körperanalysewerte. Zusätzlich dient die Anzeigevorrichtung 10 zum Anzeigen von über die Empfangseinheit 13 empfangenen Informationen. Die Anzeigevorrichtung kann als LCD ausgebildet sein und einen Touchscreen aufweisen. Mittels des Touchscreen können beispielsweise die Bedingungen zum Auslösen eines Signals durch eine Alarmerzeugungseinheit eingegeben werden. In einer alternativen Ausführungsform kann auf den Touchscreen verzichtet werden und die Bedingungen zum Auslösen eines Signals können durch nachstehend beschriebene Eingabemittel 12 eingegeben werden.

Die Anzeigevorrichtung 10 kann eine analoge und eine digitale Anzeigevorrichtung aufweisen. So kann mittels der analogen Anzeigevorrichtung das Gewicht der zu analysierenden Person mittels einem Zeigemittel und einer Skaleneinheit angezeigt werden. Es ist möglich, dass die digitale Anzeigevorrichtung derart eingestellt wird, dass sie gleichzeitig mit der analogen Anzeigevorrichtung das Gewicht der zu wiegenden Person anzeigt. Zusätzlich dient die digitale Anzeigevorrichtung zum Anzeigen der mittels der Empfangseinheit 13 empfangenen Informationen und weiterer optischer Signale. Die Körperanalysewaage 1 kann derart eingestellt werden, dass, wenn sich die Körperanalysewaage 1 nicht in einem Analysevorgang befindet, die Anzeigevorrichtung 10 das Datum und die aktuelle Zeit anzeigt.

Des Weiteren weist die Körperanalysewaage 1 Eingabemittel 12 auf, mittels denen Werte, wie z.B. das Alter, die Größe, das Geschlecht der zu analysierenden Person, etc., eingegeben werden. Diese eingegebenen Werte können beispielsweise zur genauen Ermittlung der Körperanalysewerte, wie z.B. des Körperfettanteils der zu analysierenden Person, verwendet werden.

Des Weiteren weist die Körperanalysewaage 1 mehrere in Fig. 1 nicht dargestellte Bauelemente auf. So weist die Körperanalysewaage 1 eine Alarmerzeugungseinheit auf, die ein Signal erzeugt, wenn ein voreingestellter Wert bezüglich der Körperanalysewerte ermittelt wird. Das durch die Alarmerzeugungseinheit erzeugte Signal kann optisch und/ oder akustisch wahrgenommen werden. Zusätzlich zu dem optischen und/ oder akustischen Signal kann die Alarmerzeugungseinheit bewirken, dass über die auf der Körperanalysewaage 1 angebrachte Sendeeinheit 13 eine Benachrichtigung an ein nicht auf der Körperanalysewaage 1 vorgesehenes Empfangsgerät gesendet wird. So wird diese Benachrichtigung beispielsweise als eine Kurznachricht an ein Mobiltelefon und/ oder per Funk an einen Funkempfänger gesendet.

Ferner weist die Körperanalysewaage 1 eine nicht dargestellte Speichereinheit zum Abspeichern der beispielsweise ermittelten Körperanalysewerte auf. Die Körperanalysewerte können beispielsweise mit einer Zeitangabe abgespeichert werden, so dass sichergestellt ist, dass bei einer Analyse der Daten mittels beispielsweise eines Rechners die Körperanalysewerte voneinander unterschieden werden können und eine genaue Analyse sichergestellt ist.

Des Weiteren weist die Körperanalysewaage 1 eine Steuereinheit zum Steuern der Körperanalysewaage 1, eine Spannungsmesseinrichtung zum Messen der bei einem Messvorgang über die zu analysierende Person abfallenden Spannung und eine Spannungserzeugungseinrichtung, die an das Messelement konstant eine Spannung anlegt.

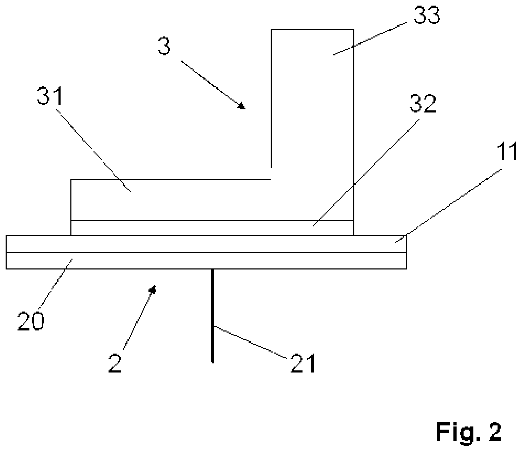

Figur 2 zeigt eine schematische Seitenansicht der Körperanalysewaage 1 und einen Teil der zu analysierenden Person 3, der mit der Körperanalysewaage 1 während des Messvorgangs in Kontakt ist. Genauer gesagt steht die zu analysierende Person mit dem Fuß 31 mit der Körperanalysewaage 1 in Kontakt. Das Messelement 2 weist eine erste Elektrode 20 auf, an die über eine Leitung 21 eine Spannung angelegt wird. Die Spannung wird durch die in der Körperanalysewaage 1 vorgesehene Spannungserzeugungseinrichtung zur Verfügung gestellt. Diese Spannungserzeugungseinrichtung ist derart ausgebildet, dass sie an die erste Elektrode 20 konstant eine Wechselspannung anlegt. Die erste Elektrode 20 ist in dieser Ausführungsform auf der von dem Fuß 31 abgewandten Seite der Kontaktplatte 11 angeordnet.

Es ist auch eine nicht dargestellte Ausführungsform vorstellbar, in der die erste Elektrode 20 innerhalb der Kontraktplatte 11 angeordnet ist. Im Ergebnis erfolgt kein direkter Kontakt der ersten Elektrode mit der zu analysierenden Person 3, weil die Kontaktplatte 11 zwischen der zu analysierenden Person 3 und der ersten Elektrode vorgesehen ist. Der Fuß 31 berührt die Kontaktplatte 11 an ihrer zu der ersten Elektrode 20 abgewandten Seite. Auf der zur Kontaktplatte 11 weisenden Seite des Fußes 31 können Verschmutzungen oder Hornhaut vorhanden sein. Anzumerken ist, dass die Körperanalysewaage 1 zwei auf der Kontaktplatte 11 angeordnete erste Elektroden 20 aufweist, wobei in Fig. 2 nur eine der beiden ersten Elektroden 20 dargestellt ist.

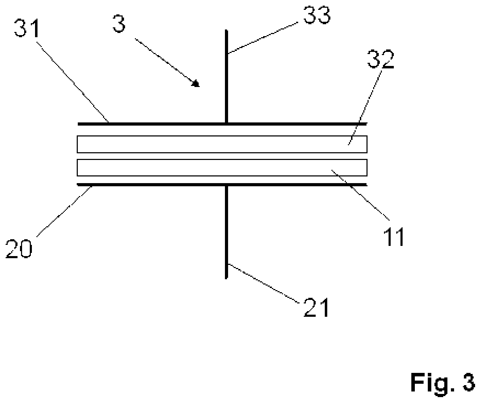

Figur 3 zeigt ein elektrisches Ersatzschaltbild der in Figur 2 gezeigten Körperanalysewaage 1. Aus den Fig. 2 und 3 ist zu entnehmen, dass in der erfindungsgemäßen Ausführungsform im Gegensatz zum Stand der Technik der Strom nicht direkt über die Elektroden in die Person eingekoppelt wird, weil die Kontaktplatte 11 zwischen der zu analysierenden Person 3 und der ersten Elektrode 20 vorgesehen ist. Eine derartige Anordnung der ersten Elektrode 20 bezüglich der zu analysierenden Person 3 entspricht einer insbesondere mit Wechselstrom beaufschlagten Hochpassschaltung, die einen Kondensator und einen Widerstand aufweist, wobei ein Spannungsabfall über den Widerstand gemessen wird.

Dabei entspricht die erste Elektrode 20 einer ersten Kondensatorplatte des Kondensators und der Fuß 31 der zweiten Elektrode bzw. der zweiten Kondensatorplatte des Kondensators. Die Kontaktplatte 11 und die Verschmutzungen bzw. die Hornhaut entsprechen dem Dielektrikum des Kondensators und wirken als ein Isolator 32, der eine deutlich geringere elektrische Leitfähigkeit als der Fuß 31 und die erste Elektrode 20 aufweist. Die zu analysierende Person 3 entspricht dem Widerstand der Hochpassschaltung, über die die abfallende Spannung gemessen wird.

Im Folgenden wird der Messvorgang zum Bestimmen der Körperanalysewerte erläutert. Sobald sich die zu analysierende Person auf die Körperanalysewaage stellt, wird durch die Spannungserzeugungseinrichtung eine positive Spannung auf eine der beiden ersten Elektroden 20 und eine negative Spannung auf die andere der beiden ersten Elektroden 20 angelegt. Die zweite Elektrode, die dem Fuß der zu analysierenden Person entspricht wird entsprechend der Ladung der ersten Elektrode 20 negativ oder positiv geladen. Als Ergebnis dieser Aufladung der zweiten Elektrode stellt sich ein Stromfluss in der zu analysierenden Person von dem einen Fuß 31 über das Bein 33 und den Unterleib zu dem anderen Bein 33 und Fuß 31 ein. Die Spannungsmesseinrichtung misst basierend auf diesem Strom die über die zu analysierende Person 3 abfallende Spannung.

Werden die beiden Kondensatoren mit Wechselstrom beaufschlagt, fließt je nach Stromstärke und Amplitudenlänge in der Regel kein oder kaum Strom durch die Person. Vielmehr wird hier das von der Unterseite des Fuß und der hautnahen Schicht des Fuß zusammen mit dem konstanten Anteil der Kontaktplatte gebildete Dielektrikum für eine Beeinflussung des Ladungsniveaus der Messelemente sorgen, die als charakteristische Größe ermittelt und zur Berechnung der Körperwerte herangezogen werden kann.

Die Steuereinheit bestimmt basierend auf der angelegten Spannung und der an der zu analysierenden Person abgefallenen Spannung eine bioelektrische Impedanz. In der Steuereinheit wird durch aus dem Stand der Technik bekannte Formeln und Faktoren unter Berücksichtigung der mittels des Eingabemittels 12 eingegebenen Werte und der ermittelten Impedanz beispielsweise der Körperfettanteil ermittelt und über die Anzeigevorrichtung ausgegeben. Die Erfindung ist nicht nur auf die Ermittlung des Körperfettanteils als Körperanalysewert beschränkt, sondern es können auch weitere Körperanalysewerte, wie z.B. die Muskelmasse bestimmt werden.

### Liste der Bezugszeichen

1 Körperanalysewaage
10 Anzeigevorrichtung
11 Kontaktplatte
12 Eingabemittel
13 Sende-/ Empfangseinheit
2 Messelement
20 erste Elektrode
21 Leitung
3 Person
31 Fuß
32 Isolator
33 Bein

## Patentansprüche

1. Körperanalysewaage zum Messen von Körperwerten, insbesondere Körperfettwerten, einer Person , mit
• einer als Wägeplatte dienenden Kontaktplatte (11), auf der die Person während einer Körperwertmessung steht,
• zwei nebeneinander angeordneten, von der Person insbesondere durch Aufstellen einer Fußsohle zu berührenden Messelementen (2) mit zwei ersten Elektroden (20) zur Messung einer zumindest mit einem Körperwert korrelierenden Größe,
• einer Auswerteelektronik, die aus der Größe den zu bestimmenden Körperwert zu berechnen vermag, und
• einer Anzeigevorrichtung zur Anzeige des berechneten Körperwerts,
**dadurch gekennzeichnet, dass**
die Kontaktplatte (11) aus einem isolierenden Material besteht oder mit einem isolierenden Material beschichtet ist,
die zwei ersten Elektroden (20) der Messelemente (2) jeweils als erste Kondensatorplatte ausgebildet und auf der Kontaktplatte (11) ohne direkten Kontakt mit der Person auf der Füßen (31) der Person abgewandten Seite der Kontaktplatte (11) oder in der Kontaktplatte (11) angeordnet sind, wobei jede der zwei ersten Elektroden zusammen mit einem als jeweilige zweite Kondensatorplatte dienenden Körperteil, insbesondere einer jeweiligen Fußsohle, der Person jeweils einen Kondensator bildet, und die Bestimmung des Körperwertes über eine Impedanzmessung erfolgt, wobei die beiden Kondensatoren innerhalb eines Stromkreises mit einem Wechselstrom beaufschlagt sind, und die Auswerteelektronik derart ausgebildet ist, dass sie ein von einer Unterseite eines jeweiligen Fußes und einer hautnahen Schicht des Fußes zusammen mit einem konstanten Anteil der Kontaktplatte gebildetes Dielektrikum zwischen der leitfähigen Schicht im Körperteil selbst und der jeweiligen ersten Kondensatorplatte zu bestimmen und hieraus die mit einem Körperwert korrelierende Größe zu berechnen mag.

2. Körperanalysewaage nach Anspruch 1, **dadurch gekennzeichnet, dass** die Wechselstromspannung derart gewählt ist, dass die Messung ohne wesentliche Stromeinleitung in die Person über die jeweilige erste Kondensatorplatte hinaus erfolgt.

3. Körperanalysewaage nach einem der vorhergehenden Ansprüche, **dadurch gekennzeichnet, dass** der Wechselstrom, mit dem die beiden Kondensatoren beaufschlagt sind, nicht direkt in den Körper der Person eingekoppelt wird, und dass eine Beeinflussung des Ladungsniveaus der von den ersten Elektroden (20) gebildeten Messelemente (2) durch das von der Unterseite eines jeweiligen Fußes (31) der Person gebildete Dielektrikum als charakteristische Größe ermittelt und zur Berechnung der Körperwerte herangezogen wird.

4. Körperanalysewaage nach einem der vorhergehenden Ansprüche, **dadurch gekennzeichnet, dass** die Körperanalysewaage ausgebildet ist zum Durchführen eines Messvorgangs, indem, sobald die Person sich auf die Körperanalysewaage stellt, eine Spannungserzeugungseinrichtung der Körperanalysewaage eine positive Spannung auf eine der zwei ersten Elektroden (20) und eine negative Spannung auf die andere der zwei ersten Elektroden (20) anlegt, so dass der eine Fuß der Person als eine zweite Elektrode entsprechend negativ geladen wird, und der andere Fuß der Person als andere zweite Elektrode entsprechend positiv geladen wird, so dass sich ein Stromfluss in der Person von dem einen Fuß über einen Unterleib der Person zu dem anderen Fuß einstellt.)

5. Körperanalysewaage nach einem der vorhergehenden Ansprüche, **dadurch gekennzeichnet, dass** sie ein Eingabemittel (12) zur Eingabe von individuellen Daten aufweist, die die Auswerteelektronik zu speichern und bei der Berechnung des Körperwertes zu berücksichtigen vermag.

6. Körperanalysewaage nach einem der vorhergehenden Ansprüche, **dadurch gekennzeichnet, dass** sie eine Sende- und Empfangseinheit (13) zur Übertragung externer Daten zur Auswertelektronik oder zur Anzeige der ermittelten Daten auf einem entfernten Anzeigemittel aufweist.

## Claims

1. Body analysis scales for measuring body values, in particular body fat values, with
• a contact plate (11) serving as a weighing platform, on which the person stands during a body value measurement,
• two measuring elements (2) arranged next to each other, to be contacted by the person in particular by placing the sole of a foot on the same, with two first electrodes (20) for measuring a dimension correlating with at least one body value,
• evaluation electronics, which can calculate the body value to be determined from the dimension, and
• a display device for displaying the calculated body value,
**characterised in that**
the contact plate (11) consists of an insulating material or is coated with an insulating material,
the two first electrodes (20) of the measuring elements (2) are each designed as a first condenser plate and are arranged on the contact plate (11) without direct contact with the person on the side of the contact plate (11) facing away from the feet (31) of the person or in the contact plate (11), wherein each of the two first electrodes each forms a condenser together with a body part serving as the respective second condenser plate, in particular the respective sole of a foot of the person, and the determination of the body value is realised by means of an impedance measurement, wherein alternating current is applied to the two condensers inside an electric circuit, and the evaluation electronics are designed in such a way that they can determine a dielectric formed between the conductive layer in the body part itself and the respective first condenser plate generated from an underside of the respective foot and a layer of the foot near the skin together with a constant proportion of the contact plate, and can calculate the dimension correlating with a body value from this.

2. Body analysis scales according to claim 1, **characterised in that** the alternating current voltage is selected in such a way that the measurement is carried out without a substantial current induction into the person through the respective condenser plate.

3. Body analysis scales according to one of the preceding claims, **characterised in that** the alternating current, which is applied to the two condensers, are not coupled directly into the body of the person, and **in that** an influencing of the charging level by the measuring elements (2) formed by the first electrodes (20) is calculated as the dielectric created by the underside of the respective foot (31) of the person as the characteristic dimension and is used for calculating the body values.

4. Body analysis scales according to one of the preceding claims, **characterised in that** the body analysis scales are designed for carrying out a measuring process **in that** a voltage generating means of the body analysis scales applies a positive voltage to one of the two first electrodes (20) and a negative voltage to the other of the two first electrodes (20) as soon as the person steps onto the body analysis scales, so that one foot of the person is correspondingly negatively charged as a second electrode, and the other foot of the person correspondingly positively charged as the other second electrode, so that a current flow is generated in the person from one foot via the lower abdomen of the person to the other foot.

5. Body analysis scales according to one of the preceding claims, **characterised in that** it has an input means (12) for entering individual data, which can store the evaluation electronics and take these into consideration when calculating the body value.

6. Body analysis scales according to one of the preceding claims, **characterised in that** they have a transmission and receiving unit (13) for transmitting external data to the evaluation electronics or for displaying the calculated data on a remote display means.

## Revendications

1. Pèse-personne impédancemètre destiné à mesurer des valeurs corporelles, notamment le taux de graisse corporelle, d'une personne, avec
• une plaque de contact (11) servant de plateau de balance sur laquelle la personne se trouve pendant la mesure des valeurs corporelles,
• deux éléments de mesure (2) disposés l'un à côté de l'autre à toucher par la personne notamment en posant une plante de pied avec deux premières électrodes (20) pour mesurer une grandeur au moins en corrélation avec une valeur corporelle,
• une électronique d'évaluation qui calcule la valeur corporelle à déterminer à partir de la grandeur, et
• un dispositif d'affichage pour afficher la valeur corporelle calculée,
**caractérisé en ce que**
la plaque de contact (11) est composée d'un matériau isolant ou recouverte d'un matériau isolant,
les deux premières électrodes (20) des éléments de mesure (2) sont respectivement formées comme une première plaque de condensateur et disposées sur la plaque de contact (11) sans contact direct avec la personne sur le côté opposé de la plaque de contact (11) aux pieds (31) de la personne ou dans la plaque de contact (11), chacune des deux premières électrodes formant respectivement un condensateur avec un membre servant respectivement de deuxième plaque de condensateur, notamment avec une plante de pied correspondante, de la personne, et une mesure d'impédance détermine la valeur corporelle, les deux condensateurs étant alimentés en courant alternatif dans un circuit électrique, et l'électronique d'évaluation est formée de telle sorte qu'elle détermine un diélectrique, constitué d'une face inférieure d'un pied correspondant et d'une couche près de la peau du pied avec une partie constante de la plaque de contact, entre la couche conductrice dans le membre lui-même et la première plaque de condensateur correspondante et calcule à partir de là la grandeur en corrélation avec une valeur corporelle.

2. Pèse-personne impédancemètre selon la revendication 1, **caractérisé en ce que** la tension de courant alternatif est choisie de telle sorte que la mesure s'effectue par la première plaque de condensateur correspondant sans trop introduire du courant dans la personne.

3. Pèse-personne impédancemètre selon l'une des revendications précédentes, **caractérisé en ce que** le courant alternatif, avec lequel les deux condensateurs sont alimentés, n'est pas conduit directement dans le corps de la personne, et **en ce qu'**une influence du niveau de charge des éléments de mesure (2) formés des premières électrodes (20) est établie par le diélectrique constitué de la face inférieure d'un pied (31) correspondant de la personne comme grandeur caractéristique et retenue pour le calcul des valeurs corporelles.

4. Pèse-personne impédancemètre selon l'une des revendications précédentes, **caractérisé en ce que** le pèse-personne impédancemètre est formé pour effectuer une prise de mesure pendant laquelle, dès que la personne se met sur le pèse-personne impédancemètre, un dispositif de génération de tension du pèse-personne impédancemètre applique une tension positive sur une des deux premières électrodes (20) et une tension négative sur l'autre des deux premières électrodes (20) de telle sorte qu'un pied de la personne est chargé négativement comme une deuxième électrode et l'autre pied de la personne est chargé positivement comme autre deuxième électrode de telle sorte qu'un flux de courant se règle dans la personne de l'un pied à l'autre pied en passant par l'abdomen de la personne.

5. Pèse-personne impédancemètre selon l'une des revendications précédentes, **caractérisé en ce qu'**il présente un moyen d'entrée (12) pour entrer des données individuelles que l'électronique d'évaluation enregistre et prend en compte lors du calcul de la valeur corporelle.

6. Pèse-personne impédancemètre selon l'une des revendications précédentes, **caractérisé en ce qu'**il présente une unité émettrice-réceptrice (13) destinée à la transmission de données externes à l'électronique d'évaluation ou pour l'affichage des données établies sur un moyen d'affichage à distance.
